# EUROPÄISCHE PATENTANMELDUNG

(11) **EP 2 873 403 A1**
(43) Veröffentlichungstag der Anmeldung: **20.05.2015**
(21) Anmeldenummer: 13005413.3
(22) Anmeldetag: 18.11.2013
(51) Int. Cl.: A61G 12/00

(54) **Stativvorrichtung mit Kollisionsüberwachung und Verfahren zur Kollisionsüberwachung**

(71) Anmelder: Ondal Medical Systems GmbH, 36088 Hünfeld (DE)
(72) Erfinder: Perplies, Stefan, 36088 Hünfeld (DE); Füg, Volker, 36043 Fulda (DE); Volkenand, Kai, 36088 Hünfeld (DE); Ickler, Fritz, 36275 Kirchheim (DE)
(74) Vertreter: Graf von Stosch, Andreas

(57) **Zusammenfassung**

Die Erfindung betrifft eine Stativvorrichtung (1) zur Anordnung in einem Operationssaal und zum örtlichen Verlagern einer medizintechnischen Einrichtung (20) im Operationssaal, umfassend die medizintechnische Einrichtung (20) sowie ein Tragsystem (10) umfassend eine Montageeinrichtung (11) und mindestens einen daran bewegbar, insbesondere schwenkbar in einem Drehgelenk (12.1, 12.2, 12.3), gelagerten Tragarm (13, 14), wobei die medizintechnische Einrichtung am Tragarm befestigt ist und in einem Aktionsradius entsprechend dem Bewegungsfreiheitsgrad des Tragsystems verlagerbar ist, und wobei die Stativvorrichtung (1) eingerichtet ist, mindestens ein Hindernis im Aktionsradius der Stativvorrichtung zu erfassen und eine mögliche Kollision mit dem Hindernis anzuzeigen und/oder zu verhindern. Ferner betrifft die Erfindung ein Verfahren zum Überwachen der Stativvorrichtung (1).

## Beschreibung

Die vorliegende Erfindung betrifft eine Stativvorrichtung zur Anordnung in einem Operationssaal und zum örtlichen Verlagern einer medizintechnischen Einrichtung im Operationssaal, welche die medizintechnische Einrichtung sowie ein Tragsystem mit mindestens einem daran bewegbar gelagerten Tragarm umfasst. Ferner betrifft die Erfindung ein Verfahren zum Überwachen einer in einem Operationssaal angeordneten Stativvorrichtung hinsichtlich einer Kollision. Die Erfindung betrifft insbesondere eine Vorrichtung mit einzelnen Merkmalen des Anspruchs 1 und insbesondere ein Verfahren mit einzelnen Merkmalen des unabhängigen Verfahrensanspruchs.

Versorgungseinheiten in Operationssälen bzw. auf Intensivstationen, insbesondere Deckenversorgungseinheiten, weisen häufig ein in der Höhe starres oder ein höhenverstellbares Stativ mit einem oder mehreren Tragarmen auf, welche jeweils um eine insbesondere vertikal ausgerichtete Achse geschwenkt und/oder translatorisch verlagert werden können, um eine am Stativ angeordnete medizintechnische Einrichtung in einer gewünschten Position zu positionieren. Um zu vermeiden, dass eine Versorgungseinheit mit weiteren im Operationssaal angeordneten Hindernissen (Personen oder Objekte oder Raumwände) kollidiert, oder dass mehrere nebeneinander angeordnete Versorgungseinheiten miteinander kollidieren, können die Versorgungseinheiten Anschläge aufweisen, welche eine Bewegung in bestimmten Richtungen begrenzen. Solche Anschläge können aber in vielen Fällen eine Kollision nicht gänzlich ausschließen, denn sie sind meist an vorbestimmten Positionen angeordnet, welche die relative Anordnung der Versorgungseinheiten zueinander oder zu weiteren Komponenten bzw. Hindernissen nicht berücksichtigen. Dadurch wird die Handhabung der Versorgungseinheiten für einen Bediener erschwert. Insbesondere muss ein Bediener eine Verlagerung besonders langsam oder vorsichtig ausführen, also mit hoher Aufmerksamkeit oder auch zeitintensiv, speziell bei Versorgungseinheiten mit einer Vielzahl von Drehgelenken oder Armen und Verstellmöglichkeiten, z.B. auch in der Höhe oder translatorisch zur Seite. Bei mehreren Armen kann die Gefahr einer Kollision dabei in Bezug auf jeden der Arme bestehen.

Es ist eine Aufgabe der vorliegenden Erfindung, eine Stativvorrichtung bereitzustellen, mittels welcher die Handhabung der Stativvorrichtung, insbesondere das Positionieren einer medizintechnischen Einrichtung der Stativvorrichtung, erleichtert werden kann.

Die Aufgabe wird durch eine Stativvorrichtung, insbesondere Deckenstativvorrichtung, mit den Merkmalen des Anspruchs 1 gelöst. Diese Stativvorrichtung zur Anordnung in einem Operationssaal und zum örtlichen Verlagern einer medizintechnischen Einrichtung im Operationssaal, umfasst die medizintechnische Einrichtung sowie ein Tragsystem, umfassend eine Montageeinrichtung, insbesondere zur Deckenmontage, und mindestens einen daran bewegbar, insbesondere schwenkbar in einem Drehgelenk, gelagerten Tragarm, wobei die medizintechnische Einrichtung am Tragarm befestigt ist und in einem Aktionsradius entsprechend dem Bewegungsfreiheitsgrad des Tragsystems verlagerbar ist, und wobei die Stativvorrichtung eingerichtet ist, mindestens ein Hindernis im Aktionsradius der Stativvorrichtung zu erfassen und eine mögliche Kollision mit dem Hindernis anzuzeigen und/oder zu verhindern. Hierdurch kann die Gefahr einer Kollision erkannt werden, und der Bediener kann auf diese Gefahr hingewiesen werden. Der vom Bediener aufzubringende Grad an Aufmerksamkeit beim Verlagern der medizintechnischen Einrichtung kann dadurch gesenkt werden. Dabei kann die Stativvorrichtung mit einem variablen Bewegungsbereich bereitgestellt werden. Der Bewegungsfreiheitsgrad ist nicht durch irgendwelche Anschläge beschränkt, welche vorgegebene Endpositionen definieren würden und eine Bewegung über beispielsweise einen Rotationswinkel hinaus blockieren würden. Vielmehr kann der größtmögliche Bewegungsfreiheitsgrad sichergestellt werden. Dabei kann die Stativvorrichtung z.B. an einer Zimmerdecke oder auch an einer Seitenwand montiert sein.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Montageeinrichtung ist dabei bevorzugt ein Flansch oder irgendeine andere Schnittstelle zu verstehen, womit das Tragsystem an einer zumindest annähernd horizontal ausgerichteten Zimmerdecke oder auch einer zumindest annähernd vertikal ausgerichteten Wand montiert werden kann. Mit anderen Worten betrifft die vorliegende Erfindung auch Stativvorrichtungen, welche alternativ oder zusätzlich an vertikalen Wänden montiert werden können. Auch eine an einer vertikalen Wand gelagerte medizintechnische Einrichtung kann Teil der erfindungsgemäßen Stativvorrichtung sein.

Als Tragarm ist dabei bevorzugt ein Ausleger oder Träger zu verstehen, welcher sich in einer bestimmten Richtung erstreckt und den gewünschten Aktionsradius für die unterschiedlichen Soll-Positionen der medizintechnischen Einrichtung sicherstellen kann, insbesondere durch eine Drehbewegung um ein Drehgelenk. Der Tragarm kann auch eine teleskopische Vorrichtung mit einem (zusätzlichen) Bewegungsfreiheitsgrad in translatorischer Richtung entlang der Längsachse des Tragarms sein.

Der Bewegungsfreiheitsgrad des Tragsystems bzw. der medizintechnischen Einrichtung kann mehrere Freiheitsgrade umfassen, also z.B. translatorische und/oder rotatorische Freiheitsgrade in mehreren Ebenen bzw. um mehrere Achsen.

Gemäß einem Ausführungsbeispiel weist die Stativvorrichtung mindestens eine Sensoreinrichtung zum Erfassen der Relativposition des mindestens einen Tragarms und/oder der medizintechnischen Einrichtung sowie eine mit der mindestens einen Sensoreinrichtung verbundene Steuerungseinrichtung zum Auswerten der erfassten Relativposition auf. Der Aktionsradius bzw. die entsprechenden Positionsdaten können dabei in einem Datenspeicher der Steuerungseinrichtung hinterlegt sein.

Als Sensoreinrichtung ist dabei bevorzugt ein Umgebungssensor mit einem oder mehreren Detektoren zu verstehen, welcher eingerichtet ist, die Umgebung, insbesondere das Vorhandensein von Gegenständen oder Personen im Aktionsradius, zu erfassen. Bevorzugt ist die Sensoreinrichtung oder mindestens eine der Sensoreinrichtungen eingerichtet, auch eine Bewegung der Stativvorrichtung zu erfassen. Hierbei kann die entsprechende Sensoreinrichtung auch mit einem Bewegungsdetektor zum Erfassen einer Bewegung der Stativvorrichtung verbunden sein oder diesen aufweisen, und der Bewegungsdetektor ist bevorzugt mit einem (Dreh-)Gelenk der Stativvorrichtung verbunden oder darin angeordnet.

Bevorzugt ist die Sensoreinrichtung eingerichtet, einen Abstand und/oder einen Winkel zu einem Hindernis zu erfassen und ein entsprechendes Signal an die Steuerungseinrichtung auszugeben. Bevorzugt ist die Sensoreinrichtung eingerichtet, den Winkel von auf die Sensoreinrichtung einfallender Strahlung zu erfassen. Hierdurch kann die Sensoreinrichtung zum Bestimmen der Relativposition über Triangulation verwendet werden. Gemäß einer Variante ist die Sensoreinrichtung eingerichtet, Strahlung mit einer modulierten Frequenz auszusenden und zu erfassen. Hierdurch kann Interferenz bei Triangulation vermieden werden.

Die Steuerungseinrichtung ist bevorzugt eingerichtet, eine mögliche Kollision in Abhängigkeit des Abstands anzuzeigen bzw. anzeigen zu lassen, insbesondere mit steigender Intensität (z.B. Helligkeit, Lautstärke) bei abnehmendem Abstand. Gemäß einer Variante kann das Anzeigen einer möglichen Kollision mit einer ersten Intensität erstmalig z.B. bei einem Abstand von 30 cm erfolgen, und mit einer zweiten Intensität (insbesondere größeren Lautstärke) ab einem Abstand von z.B. nur noch 15 cm, und mit einer dritten Intensität (insbesondere noch größeren Lautstärke) ab einem Abstand von z.B. nur noch 5 cm.

Als Hindernisse sind dabei bevorzugt irgendwelche Gegenstände oder Personen in der unmittelbaren Umgebung zu verstehen, insbesondere solche, die innerhalb vom Aktionsradius der Stativvorrichtung angeordnet sind.

Als Relativposition ist dabei bevorzugt eine Anordnung eines bewegbaren Tragarms der Stativvorrichtung relativ zur Umgebung, insbesondere relativ zu möglichen Hindernissen innerhalb des Aktionsradius, zu verstehen. Die Relativposition kann z.B. durch Entfernungsdaten zu einem potentiellen Hindernis beschrieben werden, also z.B. durch ein Signal der Sensoreinrichtung, dass in 1 Meter Entfernung ein Hindernis vorliegt, mit welchem die Stativvorrichtung kollidieren könnte, insbesondere in Abhängigkeit einer aktuellen Bewegung bzw. Bewegungsrichtung der Stativvorrichtung.

Als Steuerungseinrichtung ist dabei bevorzugt irgendein Steuerungsgerät zu verstehen, welches mit der Anzeigeeinrichtung verbunden ist und eingerichtet ist, einen potentiellen Kollisionszustand zu identifizieren, insbesondere auf Grundlage eines Abstandes zu potentiellen Hindernissen und einer Bewegung bzw. Bewegungsrichtung und/oder Bewegungsgeschwindigkeit, und mittels der Anzeigeeinrichtung anzeigen zu lassen. Die Steuerungseinrichtung umfasst bevorzugt eine Auswerteeinheit mit einem Prozessor, insbesondere einem Mikroprozessor, zum Auswerten der von dem Sensor bzw. den Sensoren erhaltenen Signale. Die Auswerteeinheit kann auf einer Steuerplatine angeordnet sein. Die Auswerteeinheit kann z.B. einen Soll-Ist-Vergleich eines erfassten Abstandes zu einem Hindernis mit einem einzuhaltenden Mindestabstand vornehmen. Die Auswerteeinheit kann auch z.B. eine Bewegungsgeschwindigkeit eines einzelnen Tragarms oder der medizintechnischen Einrichtung in Bezug auf einen aktuell vorliegenden Abstand zu einem Hindernis auswerten und ein Signal ausgeben, welches darauf hinweist, dass die Bewegung der Stativvorrichtung entweder verlangsamt werden sollte oder in eine andere Richtung umgelenkt werden sollte.

Die Steuerungseinrichtung ist dabei bevorzugt eingerichtet, die Ist-Position eines Tragarms bzw. der medizintechnischen Einrichtung relativ zum Aktionsradius der Stativvorrichtung zu bestimmen und auszuwerten, wie weit der entsprechende Tragarm oder die medizintechnische Einrichtung noch in eine bestimmte Richtung verlagert werden kann, bis eine Grenze des Aktionsradius erreicht wird. Basierend auf diesen Abstandsdaten kann dann ein einzelnes Signal einer entsprechenden Sensoreinrichtung ausgewertet werden, und es kann bestimmt werden, mit welchen vermeintlichen Hindernissen eine Kollision gar nicht möglich ist. Hierdurch kann vermieden werden, dass die Stativvorrichtung überflüssige Warnhinweise anzeigt.

Gemäß einem Ausführungsbeispiel ist die Steuerungseinrichtung mit einer Anzeigeeinrichtung zum Anzeigen einer zu einer Kollision mit den Hindernissen führenden Relativbewegung (insbesondere in Abhängigkeit der erfassten Relativposition) verbunden. Als Anzeigeeinrichtung ist dabei bevorzugt eine Warnleuchte oder ein Display oder ein Lautsprecher oder eine Einrichtung zum Erzeugen eines haptischen Signals, insbesondere eine vibrierfähige Einrichtung, zu verstehen. Die Anzeigeeinrichtung umfasst bevorzugt mindestens ein Ausgabeelement bzw. mindestens eine Signalisierungseinheit. Die Anzeigeeinrichtung kann auch mehrere dieser beispielhaft aufgeführten Einrichtungen aufweisen. Bevorzugt sind die Ausgabeelemente zumindest teilweise in Drehgelenken der Stativvorrichtung angeordnet, so dass einem Bediener angezeigt werden kann, an welchem Drehgelenk eine weitere Bewegung zu einer Kollision führen würde.

Als ein Anzeigen ist dabei bevorzugt allgemein ein Kenntlichmachen einer Kollisionsgefahr bzw. irgendein Hinweis darauf zu verstehen, insbesondere für einen Bediener der medizintechnischen Einrichtung. Das Anzeigen erfordert also nicht notwendigerweise ein visuelles Signal.

Als eine zu einer Kollision führende Relativbewegung ist dabei bevorzugt eine Bewegung mindestens eines Tragarms zu verstehen, bei welcher eine Kollision mit einem Hindernis der Umgebung nicht vermieden werden kann, sofern die Bewegung in derselben Weise fortgeführt wird.

Eine Verbindung bzw. ein "verbunden sein" mit der Steuerungseinrichtung kann dabei durch eine drahtgebundene oder drahtlose Leitung erfolgen.

Gemäß einem Ausführungsbeispiel weist die Stativvorrichtung mindestens eine insbesondere im mindestens einen Drehgelenk angeordnete Bremseinrichtung auf, welche mit der Steuerungseinrichtung verbunden ist, und welche mittels der Steuerungseinrichtung (insbesondere in Abhängigkeit der erfassten Relativposition) derart ansteuerbar ist, dass eine Bewegung des Tragsystems bei einer zu einer Kollision führenden Relativbewegung zumindest teilweise blockierbar ist. Hierdurch kann ein Eingriff in den Bedienablauf vorgenommen werden, und eine Kollision kann aktiv verhindert werden. Als Bremseinrichtung ist dabei bevorzugt eine einzelne Bremse oder eine Mehrzahl von Bremsen zu verstehen, welche z.B. mechanischer, elektrischer, hydraulischer oder magnetischer Art sind und jeweils mit der Steuerungsvorrichtung verbunden sind. Bevorzugt ist die Steuerungseinrichtung eingerichtet, die Bremseinrichtung derart anzusteuern, dass eine Bremse der Bremseinrichtung eine vorbestimmte Bremskraft ausübt. Die Steuerungseinrichtung ist dann eingerichtet, eine vorbestimmte Bremskraft einzustellen, und die Bremse ist eingerichtet, eine vorbestimmte Bremskraft auszuüben. Hierdurch können einzelne Bremsen in einzelnen Gelenken wirken, ohne dass die Stativvorrichtung vollständig aufgestoppt werden muss. Die Verlagerungsrichtung einzelner Tragarme kann dadurch beeinflusst werden, ohne dass der Behandlungsablauf eines Operateurs gestört wird. Denn gerade bei einer Vielzahl von Tragarmen kann eine bestimmte Soll-Position auf unterschiedliche Weise, also bei unterschiedlicher relativer Anordnung der einzelnen Tragarme zueinander erreicht werden. Hierdurch wird die Handhabung weiter erleichtert, und ein automatischer Eingriff während der manuellen Handhabung kann auf zweckdienliche Weise erfolgen.

Gemäß einer Variante ist die Steuerungseinrichtung eingerichtet, eine Bremskraft in Abhängigkeit eines erfassten Abstands zum Hindernis einzustellen, insbesondere graduell steigend mit abnehmendem Abstand. Gemäß einer Variante kann das Bremsen mit einer ersten Intensität erstmalig z.B. bei einem Abstand von 30 cm erfolgen, und mit einer zweiten Intensität (insbesondere stärkeren Bremskraft) ab einem Abstand von z.B. nur noch 15 cm, und mit einer dritten Intensität (insbesondere noch stärkeren Bremskraft) ab einem Abstand von z.B. nur noch 5 cm. Die Bremskraft kann dabei auch so eingestellt werden, dass die Stativvorrichtung ab einem bestimmten, vordefinierten Abstand aufgestoppt wird und zum Stillstand gebracht wird.

Gemäß einem Ausführungsbeispiel weist die Stativvorrichtung mindestens eine insbesondere mit dem mindestens einen Drehgelenk gekoppelte Antriebseinrichtung auf, welche mit der Steuerungseinrichtung verbunden ist, und welche mittels der Steuerungseinrichtung (insbesondere in Abhängigkeit der erfassten Relativposition) derart ansteuerbar ist, dass eine Bewegung des Tragsystems bei einer zu einer Kollision führenden Relativbewegung beeinflussbar ist. Hierdurch kann ein Eingriff in den Bedienablauf vorgenommen werden, und eine Kollision kann aktiv verhindert werden. Mittels der Antriebseinrichtung kann insbesondere ein Aufstoppen erfolgen, wobei die Antriebseinrichtung oder zumindest eine einzelne Antriebseinheit der Antriebseinrichtung abgeschaltet werden kann.

Eine Kopplung bzw. ein "gekoppelt sein" kann dabei als eine Wirkverbindung verstanden werden, insbesondere eine Verbindung, über welche eine Linearkraft und/oder ein Drehmoment übertragen werden kann.

Gemäß einer Variante ist die Antriebseinrichtung mittels der Steuerungseinrichtung in Abhängigkeit der erfassten Relativposition derart ansteuerbar, dass das Tragsystem bei einer zu einer Kollision führenden Relativbewegung zumindest teilweise motorisch verlagert wird, insbesondere durch Anlegen eines Drehmomentes im mindestens einen Drehgelenk. Hierdurch kann ein Eingriff in den Bedienablauf vorgenommen werden, und eine Kollision kann aktiv verhindert werden, ohne dass die Stativvorrichtung abgestoppt und neu beschleunigt werden muss. Mit anderen Worten kann die Antriebseinrichtung einen Bediener unterstützen, die medizintechnischen Einrichtung in die Soll-Position zu verlagern. Bei diesem Ausführungsbeispiel ist es möglich, die medizintechnischen Einrichtung mit geringer Kraft oder geringer Aufmerksamkeit, z.B. nur mit einer Hand, zu verlagern.

Als Antriebseinrichtung ist dabei bevorzugt eine einzelne oder mehrere Antriebseinheiten, wie z.B. ein Drehantrieb oder ein translatorischer Antrieb (Linearantrieb), zu verstehen, wobei die Antriebseinheiten jeweils in Gelenken, insbesondere Drehgelenken der Stativvorrichtung angeordnet sind oder zumindest auf diese Gelenke einwirken. Als Gelenk ist dabei ein Gelenk im weiteren Sinne zu verstehen und es kann darunter auch z.B. ein Axiallager zu verstehen sein. Es muss sich also nicht notwendigerweise im engeren Sinne um ein Drehgelenk handeln.

Gemäß einem Ausführungsbeispiel weist die Stativvorrichtung eine Mehrzahl von Sensoreinrichtungen auf, wobei die Sensoreinrichtungen am mindestens einen Tragarm und/oder an der medizintechnischen Einrichtung angeordnet sind. Hierdurch kann eine Kollisionsüberwachung in jeder beliebigen Lage und Ausrichtung der Stativvorrichtung erfolgen, insbesondere auf sehr sichere und zuverlässige Weise. Eine größere Anzahl von Sensoreinrichtungen kann sicherstellen, dass auch kleine Hindernisse bzw. deren relative Position erfasst werden können, insbesondere bei Triangulation.

Gemäß einem Ausführungsbeispiel weist die Stativvorrichtung mindestens eine Sensoreinrichtung aus der Gruppe umfassend die folgenden Sensoreinrichtungen auf: Infrarotsensor, Ultraschallsensor, kapazitiver Sensor, induktiver Sensor, Radarsensor. Bevorzugt weist die Stativvorrichtung mehrere Sensoreinrichtungen auf, insbesondere zumindest zwei unterschiedliche Sensoreinrichtungen mit voneinander abweichenden Messprinzipien. Bevorzugt weist die Stativvorrichtung mindestens zwei voneinander unterschiedliche Sensoreinrichtungen aus der Gruppe umfassend die folgenden Sensortypen auf: Infrarotsensoren, Ultraschallsensoren, kapazitive Sensoren, induktive Sensoren, Radarsensoren, oder Beschleunigungssensoren. Bevorzugt weist die Stativvorrichtung mehrere Sensoreinrichtungen mit unterschiedlichen Erfassungsbereichen und unterschiedlichen Wirkprinzipien auf. Hierdurch kann die Erfassung auf individualisierte Weise erfolgen. Es können die an jeweiligen Montage-Positionen der Stativvorrichtung jeweils am besten geeigneten Sensoren verwende werden, je nach Größe des zu überwachenden Bereichs oder Art der Gegenstände, mit welchen eine Kollision nicht ausgeschlossen werden kann. Eine Montage kann dabei z.B. durch Anschrauben, Ankleben, oder Einclipsen. Auch kann die Stativvorrichtung eine verstellbare Aufnahme zur Anordnung von einem oder mehreren Sensoren aufweisen.

Gemäß einem Ausführungsbeispiel weist die Stativvorrichtung mindestens zwei Tragarme auf, an welchen jeweils eine Mehrzahl von Sensoreinrichtungen, insbesondere zwei, drei oder vier Sensoreinrichtungen, angeordnet sind, bevorzugt beidseitig und gegenüberliegend an Seitenflächen des jeweiligen Tragarms. Dies ermöglicht bei schwenkbar bzw. drehbar um eine Achse gelagerten Tragarmen eine Erfassung irgendwelcher Hindernisse in beiden Schwenkrichtungen. Bei mehreren Tragarmen sind die Sensoreinrichtungen bevorzugt an allen Tragarmen angeordnet.

Gemäß einer Variante sind an mindestens einem Tragarm eine oder mehrere Sensoreinrichtungen des Typs Infrarotsensor angeordnet, und die Infrarotsensoren weisen jeweils eine Leuchtdiode (LED) auf, welche eingerichtet ist, Infrarotstrahlung auszustrahlen, sowie einen Detektor, insbesondere einen so genannten Position Sensitive Detector (PSD), welcher eingerichtet ist, Infrarotstrahlung zu erfassen. Bei dieser Variante ist die Steuerungseinrichtung bevorzugt eingerichtet, die Relativposition durch Triangulation auszuwerten. Bei der Triangulation kann eine Abstandsmessung in Bezug auf ein Hindernis erfolgen, indem Messwerte von einer Mehrzahl von Sensoreinrichtungen ausgewertet werden. Beispielsweise kann eine Triangulation mit Infrarotsensoren durchgeführt werden, welche Strahlung aussenden und die reflektierte Strahlung auswerten oder zumindest erfassen. Dabei kann der Winkel der einfallenden Strahlung erfasst werden, und über den Winkel kann die Position des Hindernisses ermittelt werden.

Bevorzugt weist die Stativvorrichtung mehrere Sensoreinrichtungen auf, welche in einem vorbestimmten Abstand zueinander angeordnet sind, insbesondere am mindestens einen Tragarm. Der Abstand kann z.B. im Bereich von 15-20 cm gewählt werden. Weiter bevorzugt sind die Sensoreinrichtungen am Tragarm entlang der kompletten Längserstreckung des Tragarms derart angeordnet, dass ein Mindestabstand zwischen zwei Sensoreinrichtungen bzw. zu einem freien Ende des Tragarms nicht unterschritten wird. Der Mindestabstand zu einem freien Ende kann dabei auch in Abhängigkeit eines Erfassungsbereichs der Sensoreinrichtung gewählt werden. Der Mindestabstand liegt bevorzugt im Bereich von 10-20 cm.

Gemäß einer Variante ist die Sensoreinrichtung derart an der Stativvorrichtung angeordnet, dass ein Detektor, insbesondere ein Objektiv oder eine Linse, der Sensoreinrichtung in Bezug auf eine Außenoberfläche des entsprechenden Tragarms bzw. der medizintechnischen Einrichtung hervorsteht. Die hervorstehende Anordnung kann sicherstellen, dass die Sensoreinrichtung einen großen Bereich erfassen kann, also z.B. einen Kegel mit einem großen Öffnungswinkel von z.B. 70° bis 90°.

Gemäß einer Variante ist die Sensoreinrichtung eingerichtet, einen Erfassungsbereich zu überwachen, welcher kleiner oder gleich dem Aktionsradius der Stativvorrichtung ist. Hierdurch kann vermieden werden, dass die Steuerungsvorrichtung auch in einem Fall ein Warnsignal ausgibt oder in einen Bedienablauf eingreift, in welchem gar keine Kollision möglich ist. Insbesondere kann vermieden werden, dass die Sensoreinrichtung ein Signal in Hinblick auf eine Komponente (ein vermeintliches Hindernis) ausgibt, welche gar nicht im Aktionsradius liegt.

Gemäß einem Ausführungsbeispiel ist jeweils mindestens eine Sensoreinrichtung am mindestens einen Tragarm angeordnet, wobei der jeweilige Tragarm zumindest schwenkbar gelagert ist, und wobei die Sensoreinrichtung eingerichtet ist, einen Erfassungsbereich zu überwachen, welcher in einer Erstreckungsebene des Tragarms einen größeren Erfassungswinkel aufweist als in einer Ebene senkrecht zur Erstreckungsebene. Hierdurch kann vermieden werden, dass bei Stativvorrichtungen mit mehreren sich überlappenden Tragarmen einer der Tragarme von einer der Sensoreinrichtungen, insbesondere von einer an einem weiteren der Tragarme angeordneten Sensoreinrichtung, fälschlicherweise als Hindernis identifiziert wird. Vielmehr können einzelne Tragarme frei zueinander verschwenkt werden, ohne dass sie als Hindernis identifiziert werden.

Als Erfassungswinkel ist dabei bevorzugt ein Winkel zu verstehen, welcher in Bezug auf eine bestimmte Raumachse den Raumbereich beschreibt, in welchem die Sensoreinrichtung Hindernisse bzw. Komponenten erfassen kann. Der Erfassungswinkel muss nicht notwendigerweise einem Öffnungswinkel eines Kegels entsprechen. Vielmehr kann der Erfassungsbereich durch mindestens zwei unterschiedliche Erfassungswinkel gekennzeichnet sein. Am Beispiel einer streng horizontalen Ausrichtung des Tragarms kann der Erfassungsbereich durch einen Vertikalwinkel (entsprechend der Summe aus einem Höhenwinkel und einem Tiefenwinkel) und einem Horizontalwinkel (entsprechend einem Azimutwinkel) gekennzeichnet sein. Für diesen Fall ist die Sensoreinrichtung eingerichtet, einen Erfassungsbereich zu überwachen, der durch einen Horizontalwinkel gekennzeichnet ist, der größer ist als der Vertikalwinkel. Bevorzugt ist der Horizontalwinkel mindestens doppelt so groß wie der Vertikalwinkel. Gemäß einer Variante liegt der Vertikalwinkel im Bereich von 10° bis 80°, insbesondere im Bereich von 20° bis 70°, oder im Bereich von 35° bis 55°. Gemäß einer Variante liegt der Horizontalwinkel im Bereich von 90° bis 180°, insbesondere im Bereich von 110° bis 160°, oder im Bereich von 125° bis 145°.

Als Erstreckungsebene ist dabei bevorzugt eine Ebene zu verstehen, in welcher sich der Tragarm in der Hauptsache erstreckt, also in welcher der Tragarm die größte Längenausdehnung aufweist. Die Erstreckungsebene muss nicht notwendigerweise horizontal ausgerichtet sein. In einigen Fällen können die Tragarme nicht nur um eine vertikale Achse verschwenkt werden, sondern auch um eine horizontal ausgerichtete Achse gekippt werden, so dass die Erstreckungsebene auch in einem Winkel von z.B. 0 bis 45° zur horizontal ausgerichteten Ebene ausgerichtet sein kann.

Bevorzugt ist die Sensoreinrichtung an einer Seitenfläche des Tragarms angeordnet, also einer Fläche, welche bei einer Deckenaufhängung der Stativvorrichtung und bei einer zumindest annähernd horizontalen Ausrichtung des Tragarms zumindest annähernd in horizontaler Richtung ausgerichtet ist. Die Seitenfläche weist bevorzugt zur Seite in einer Ebene senkrecht zu einer (vertikalen) Schwenkachse des Tragarms. Die Seitenfläche weist also typischerweise nicht nach oben zur Decke oder nach unten zum Boden des Operationssaals.

Gemäß einem Ausführungsbeispiel ist die mindestens eine Sensoreinrichtung ein Infrarotsensor. Ein Infrarotsensor ist bevorzugt an einer Seitenfläche des Tragarms angeordnet und weist in unterschiedlichen Raumrichtungen bevorzugt unterschiedliche Erfassungswinkel auf. Bevorzugt weist die Stativvorrichtung mindestens zwei am mindestens einen Tragarm angeordnete Infrarotsensoren auf, die insbesondere an einer oder beiden Seiten des Tragarms angeordnet sind.

Gemäß einem Ausführungsbeispiel ist die Stativvorrichtung höhenverstellbar, wobei die mindestens eine Sensoreinrichtung an der medizintechnischen Einrichtung angeordnet ist, und wobei die Sensoreinrichtung eingerichtet ist, einen Erfassungsbereich zu überwachen, welcher kegelförmig ist, insbesondere kegelförmig mit einem Öffnungswinkel größer 45°, bevorzugt zwischen 60° und 90°, weiter bevorzugt zwischen 70° und 85°. Als Öffnungswinkel ist dabei, bevorzugt am Beispiel einer Kegelgeometrie erläutert, das Doppelte des Winkels zwischen den Mantellinien und der Achse eines Drehkegels zu verstehen. Ein derartiger Öffnungswinkel kann einen großen Erfassungsbereich sicherstellen. Es kann mittels eines einzigen Sensors ein großer Bereich erfasst werden, insbesondere gemäß dem Prinzip einer Rundumsicht-Kamera, welche den gesamten Bereich vor der Kamera in 360° überwachen kann.

Gemäß einem Ausführungsbeispiel ist die mindestens eine Sensoreinrichtung ein an einer Unterseite der medizintechnischen Einrichtung angeordneter Ultraschallsensor. Bevorzugt liegt der Öffnungswinkel des Ultraschallsensors im Bereich von 130° bis 180°, weiter bevorzugt im Bereich von 140° bis 175°, besonders bevorzugt im Bereich von 150° bis 170°. Gemäß einer Variante sind an der Unterseite mindestens zwei Ultraschallsensoren angeordnet, insbesondere versetzt zueinander, und insbesondere für den Fall dass die Unterseite uneben ist und ein einzelner Sensor den gesamten unteren Bereich nicht überwachen kann, selbst für den Fall dass er einen Öffnungswinkel von 180° oder mehr aufweist.

Gemäß einer Variante mehrere Sensoreinrichtungen vorgesehen, welche an einer Unterseite und/oder Oberseite der medizintechnischen Einrichtung bzw. der Tragarme angeordnet sind, wahlweise in Kombination mit an einer Seitenfläche der Tragarme angeordneten Sensoreinrichtungen. Hierdurch kann die Überwachung auf noch genauerer Weise erfolgen, auch bei vertikal verlagerbaren Vorrichtungen bzw. Armen.

Gemäß einem Ausführungsbeispiel weist die Stativvorrichtung eine Anzeigeeinrichtung mit mindestens einem Anzeigeelement auf, welches zumindest am Tragsystem angeordnet ist und eingerichtet ist, ein akustisches und/oder visuelles Signal auszugeben. Diese Art von Signalen können von einem Bediener auch erkannt werden, ohne dass der Bediener in Kontakt mit der medizintechnischen Einrichtung ist.

Gemäß einem Ausführungsbeispiel weist die Stativvorrichtung eine Anzeigeeinrichtung mit mindestens einem Anzeigeelement auf, welches zumindest an der medizintechnischen Einrichtung, insbesondere an einem Griff und/oder einer Taste, angeordnet ist und eingerichtet ist, ein haptisches Signal, insbesondere ein Vibrieren, auszugeben. Hierdurch kann ein Bediener auch ohne Sichtkontakt zur Stativvorrichtung oder bei einem hohen Geräuschpegel unmissverständlich darauf hingewiesen werden, dass eine Kollision droht.

Bevorzugt sind mehrere Anzeigelemente vorgesehen, welche an einem jeweiligen Tragarm bzw. an der medizintechnischen Einrichtung angeordnet sind. Weiter bevorzugt sind Anzeigeelemente an beiden jeweiligen Enden eines jeweiligen Tragarms angeordnet. Die Steuerungseinrichtung ist bevorzugt eingerichtet, die Anzeigeeinrichtung bzw. die Anzeigeelemente derart anzusteuern, dass an derjenigen Position der Stativvorrichtung ein Warnsignal ausgegeben wird, an welcher eine Kollision droht. Hierdurch kann einem Bediener eine Kollisionsgefahr noch deutlicher kenntlich gemacht werden.

Die zuvor genannte Aufgabe wird auch durch ein Verfahren mit den Merkmalen des unabhängigen Verfahrensanspruchs gelöst. Dieses Verfahren zum Überwachen einer in einem Operationssaal angeordneten Stativvorrichtung hinsichtlich einer Kollision, insbesondere einer erfindungsgemäßen Stativvorrichtung, ist durch die folgenden Schritte gekennzeichnet:
- Erfassen eines Hindernisses in einem Aktionsradius der Stativvorrichtung mittels mindestens einer Sensoreinrichtung, insbesondere Erfassen einer Relativposition mindestens eines Tragarms der Stativvorrichtung und/oder einer medizintechnischen Einrichtung der Stativvorrichtung, jeweils relativ zu weiteren im Operationssaal angeordneten Hindernissen;
- Auswerten einer Relativposition der Stativvorrichtung relativ zum Hindernis mittels einer Steuerungseinrichtung;
- Anzeigen einer zu einer Kollision mit dem Hindernis führenden Relativbewegung in Abhängigkeit der Relativposition mittels mindestens einer Anzeigeeinrichtung, insbesondere einer an der Stativvorrichtung angeordneten Anzeigeeinrichtung; und/oder
- aktives Verhindern einer Kollision mit dem Hindernis mittels der Steuerungseinrichtung, insbesondere durch Ansteuern einer Bremseinrichtung und/oder einer Antriebseinrichtung der Stativvorrichtung.

Das aktive Verhindern kann ein Ansteuern mindestens eines Antriebs einer Antriebseinrichtung oder Ansteuern mindestens einer Bremse einer Bremseinrichtung umfassen, jeweils zum Aufstoppen der Stativvorrichtung. Durch einen derartigen Eingriff in den Bedienablauf kann eine Kollision aktiv verhindert werden, insbesondere für den Fall dass der Bediener nicht schnell genug reagieren kann, z.B. weil er nur eine Hand frei hat.

Gemäß einer Variante erfolgt das Erfassen und/oder Auswerten kontinuierlich, d.h. permanent, ohne Zeitunterbrechung. Bevorzugt erfolgt das Erfassen und/oder Auswerten dann kontinuierlich, wenn eine Bewegung der Stativvorrichtung erfolgt. Hierzu kann die Stativvorrichtung Bewegungssensoren, insbesondere in den Drehgelenken angeordnete Bewegungssensoren aufweisen, welche mit der Steuerungseinrichtung verbunden sind.

In den nachfolgenden Zeichnungsfiguren wird die Erfindung anhand von Ausführungsbeispielen näher erläutert. Es zeigen:
- Figur 1: in schematischer Darstellung in einer perspektivischen Seitenansicht eine Stativvorrichtung gemäß einem Ausführungsbeispiel der Erfindung;
- Figur 2: in schematischer Darstellung in perspektivischer Ansicht von unten die in Figur 1 gezeigte Stativvorrichtung;
- Figur 3: in schematischer Darstellung in einer perspektivischen Seitenansicht eine Stativvorrichtung gemäß einem weiteren Ausführungsbeispiel;
- Figur 4: in schematischer Darstellung in einer perspektivischen Seitenansicht eine Stativvorrichtung gemäß einem weiteren Ausführungsbeispiel; und
- Figur 5: in schematischer Darstellung Verfahrensschritte eines Verfahrens gemäß einem Ausführungsbeispiel der Erfindung.

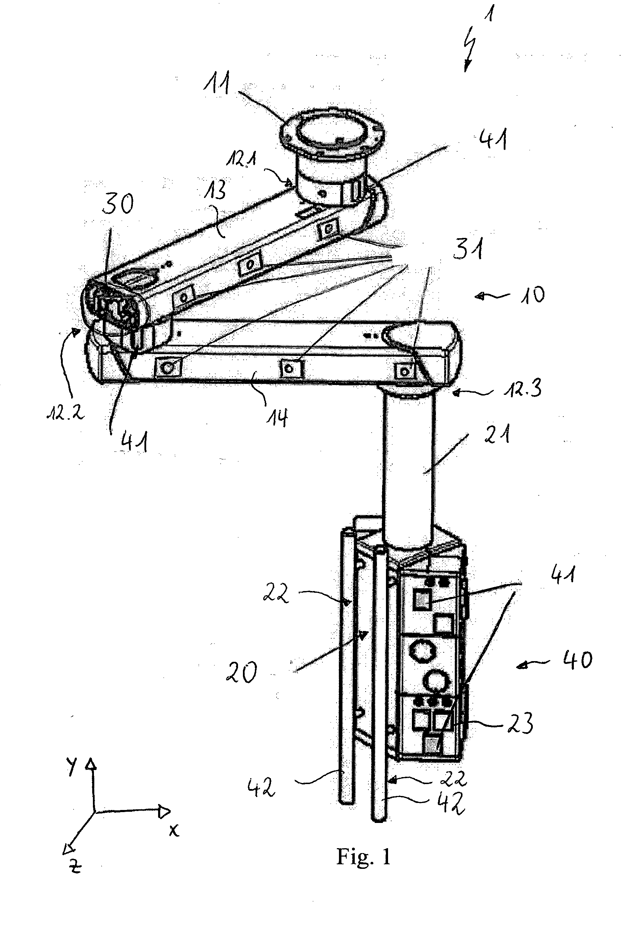

In der Figur 1 ist eine Stativvorrichtung 1 gezeigt, die ein Tragsystem 10 mit einer Montageeinrichtung 11, insbesondere einem Deckenflansch, und einem ersten Tragarm 13 sowie einem zweiten Tragarm 14 aufweist. Der erste Tragarm 13 ist am Deckenflansch 11 in einem Drehgelenk 12.1 gelagert, und der zweite Tragarm 14 ist am ersten Tragarm 13 in einem Drehgelenk 12.2 gelagert. Am Tragsystem 10 ist eine medizintechnische Einrichtung 20 angeordnet, insbesondere gelagert am zweiten Tragarm 14 in einem weiteren Drehgelenk 12.3. Die Einrichtung 20 kann als Versorgungskonsole bezeichnet werden, die mittels eines Trägers 21, insbesondere einem Konsolenrohr, am zweiten Tragarm 14 gelagert ist. Die Versorgungseinheit 20 weist zwei Griffe 22 auf, mittels welchen ein Bediener die Versorgungseinheit manuell verlagern kann. An der Versorgungskonsole 20 sind ferner Bedientasten 23 angeordnet. Die Stativvorrichtung 1 weist ferner eine Steuerungsvorrichtung 30 auf, welche im gezeigten Beispiel am ersten Tragarm 13 angeordnet ist. Die Steuerungsvorrichtung 30 ist mit mehreren Sensoreinrichtungen 31 verbunden, welche sowohl am ersten Tragarm 13 als auch am zweiten Tragarm 14 angeordnet sind, insbesondere in einem zumindest annähernd gleichen Abstand zueinander. Die Sensoreinrichtungen 31 sind an einer jeweiligen Seitenfläche des jeweiligen Tragarms angeordnet. Die Seitenflächen weisen in Richtung der X-Z-Ebene und sind zumindest annähernd in der X-Y-Ebene bzw. parallel dazu ausgerichtet. Bevorzugt handelt es sich bei den Sensoren 31 um Infrarotsensoren. Ferner weist die Stativvorrichtung 1 eine Anzeigeeinrichtung 40 auf, welche mehrere Ausgabeelemente 41, 42 umfasst. Einige der Ausgabeelemente 41 sind dabei im Bereich der Drehgelenke 12.1, 12.2, 12.3 angeordnet, und zwei Ausgabeelemente 42 sind am Griff 22 angeordnet. Die Ausgabeelemente 42 sind bevorzugt haptischer Natur, und insbesondere eingerichtet, eine Vibration am Griff 22 auszulösen. Die Ausgabeelemente 42 können z.B. als einzelne vibrierende Tasten bzw. Griffflächen ausgeführt sein. Als haptische Aktoren können z.B. Motoren eingesetzt werden, welche eine Unwucht aufweisen, oder Piezo-Scheiben. Die anderen Ausgabeelemente 41 sind bevorzugt visueller und/oder akustischer Art.

Die Stativvorrichtung 1 ist eingerichtet, mindestens ein Hindernis im Aktionsradius der Stativvorrichtung zu erfassen und einem Bediener die Möglichkeit einer Kollision mit einem Hindernis (nicht dargestellt) anzuzeigen. Hierzu können die Sensoreinrichtungen 31 einen Abstand zu einem Hindernis und/oder eine Bewegung des Tragsystems 10 bzw. der Konsole 20 erfassen, und ein entsprechendes Sensorsignal an die Steuerungsvorrichtung 30 ausgeben. Die Steuerungsvorrichtung 30 kann daraufhin auswerten, ob eine Relativposition bzw. -bewegung des Tragsystems 10 bzw. der Konsole 20 zu einer Kollision mit einem Hindernis führen könnte. Für den Fall einer vorliegenden Kollisionsgefahr kann die Steuerungsvorrichtung 30 daraufhin die Anzeigeeinrichtung 40 anweisen, an mindestens einem der Ausgabeelemente 41, 42 die Kollisionsgefahr anzuzeigen. Dies kann visuell und/oder akustisch und/oder auf haptische Weise, insbesondere durch Vibrieren, erfolgen.

In der Figur 1 ist ferner ein Koordinatensystem gezeigt, welches mit der X-Z-Ebene eine Haupterstreckungsebene der Tragarme 13, 14 andeutet. Die Sensoren 31 weisen in der X-Z-Ebene einen großen Erfassungswinkel auf, nämlich einen großen Azimut- bzw. Horizontalwinkel, und in einer vertikalen Richtung, also in Y-Richtung bzw. in einer X-Y-Ebene, bevorzugt nur einen kleinen Erfassungswinkel (kleiner Vertikalwinkel). Hierdurch kann vermieden werden, dass die am ersten Tragarm 13 angeordneten Sensoren den zweiten Tragarm 14 als Hindernis erkennen, und umgekehrt.

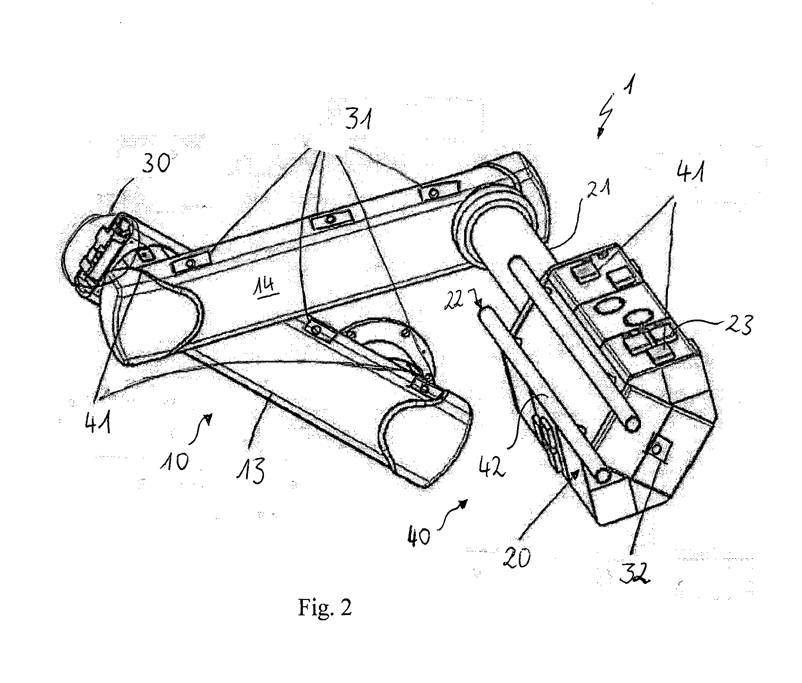

In der Figur 2 ist die Stativvorrichtung 1 von einer Unterseite her gezeigt. Es ist erkennbar, dass eine weitere Sensoreinrichtung 32 an einer Unterseite der Konsole 20 angeordnet ist. Bei dieser Sensoreinrichtung handelt es sich bevorzugt um einen Ultraschallsensor mit einem großen Erfassungswinkel, insbesondere kegelförmigen Öffnungswinkel. Auch dieser Sensor 32 ist mit der Steuerungsvorrichtung 30 verbunden und eingerichtet, ein Signal an die Steuerungsvorrichtung auszugeben, sobald ein Hindernis im Erfassungsbereich des Sensors 32 erkannt wird. Der Erfassungswinkel des Ultraschallsensors 32 kann sehr viel größer gewählt werden als derjenige der Sensoren 31 (zumindest als der Vertikalwinkel), da an der Unterseite der Konsole 20 keine weiteren Komponenten der Stativvorrichtung 1 vorhanden sind.

Im Zusammenhang mit der Beschreibung der folgenden Figuren wird bei Bezugszeichen, die nicht explizit erläutert werden, auf das Ausführungsbeispiel der Figur 1 verwiesen.

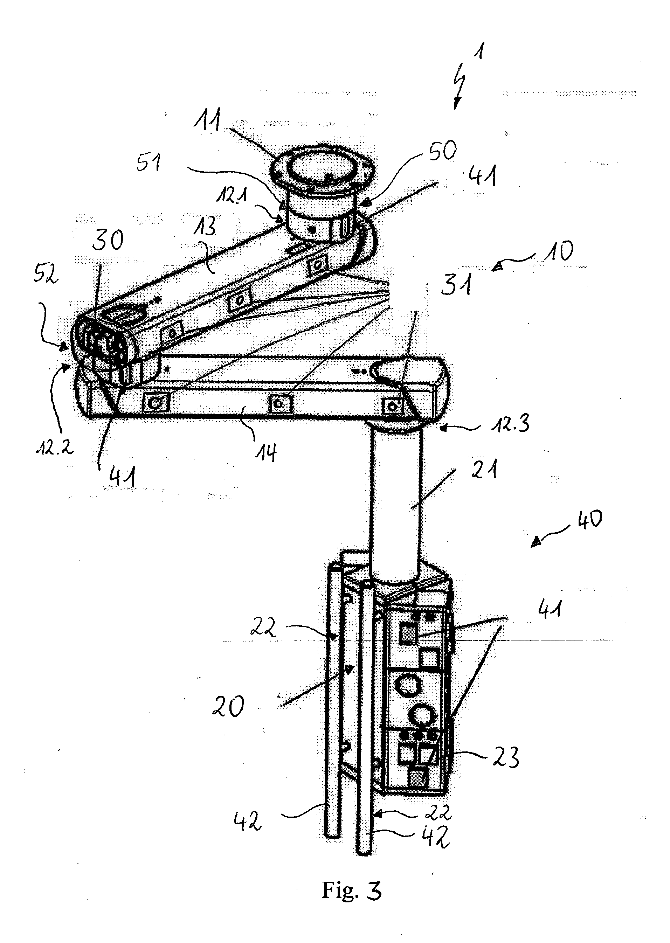

In der Figur 3 ist eine Stativvorrichtung 1 gezeigt, welche zusätzlich zu den in Figur 1 und 2 gezeigten Komponenten auch eine Bremseinrichtung 50 aufweist, die eine erste Bremse 51 und eine zweite Bremse 52 umfasst. Die Bremseinrichtung 50 ist mit der Steuerungsvorrichtung 30 verbunden, und die beiden Bremsen 51, 52 sind jeweils in einem der Drehgelenke 12.1, 12.2 angeordnet. Bei diesem Ausführungsbeispiel kann die Steuerungsvorrichtung für den Fall einer Kollisionsgefahr aktiv in den Bewegungsablauf eingreifen und die Bewegung der Stativvorrichtung 1 blockieren. Hierzu ist die Steuerungsvorrichtung eingerichtet, die erste Bremse 51 und/oder die zweite Bremse 52 zum Ausüben einer Bremskraft auf das jeweilige Gelenk anzusteuern, also das entsprechende Drehgelenk zu blockieren.

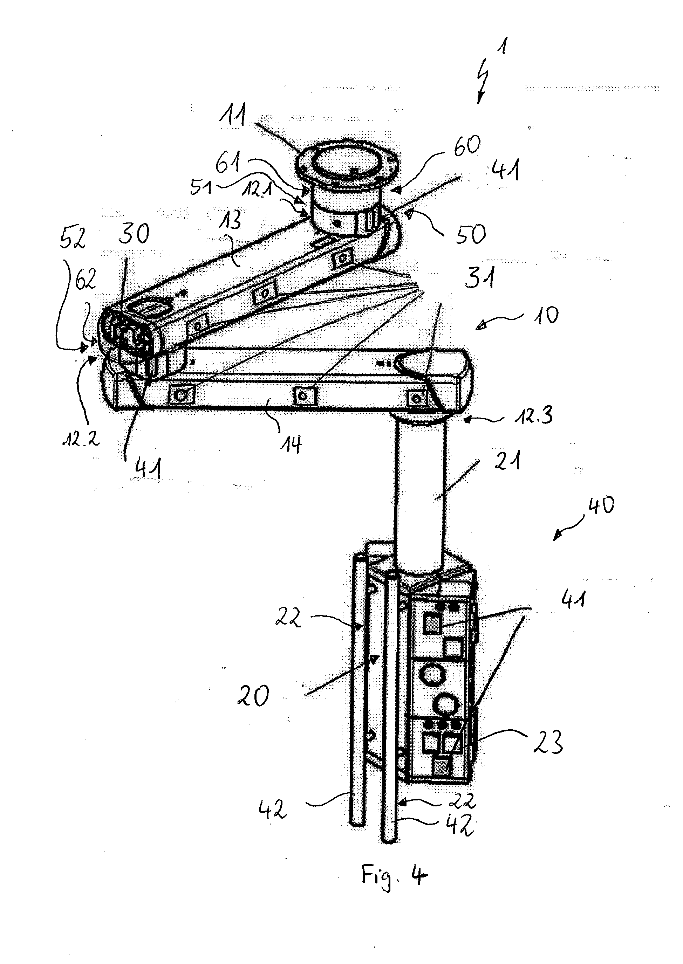

In der Figur 4 ist eine Stativvorrichtung 1 gezeigt, welche zusätzlich zu den in der Figur 3 gezeigten Komponenten auch eine Antriebseinrichtung 60 aufweist, welche einen ersten Drehantrieb 61 und einen zweiten Drehantrieb 62 umfasst. Die Antriebseinrichtung ist mit der Steuerungsvorrichtung 30 verbunden und eingerichtet, eine Bewegung des Tragsystems 10 zu bewirken. Mit anderen Worten handelt es sich bei der Stativvorrichtung 1 um eine motorisch verlagerbare Stativvorrichtung 1. In Reaktion auf eine mittels der Steuerungsvorrichtung 30 erkannte Kollisionsgefahr kann die Antriebseinrichtung 60 abgestellt bzw. gestoppt werden, so dass eine motorische Verlagerung des Tragsystems 10 unterbunden wird. Wahlweise kann die Antriebseinrichtung 60 auch derart angesteuert werden, dass ein Hindernis aktiv umfahren wird. Hierdurch kann vermieden werden, dass die Stativvorrichtung 1 komplett aufgestoppt wird. Diese Variante ist besonders bedienerfreundlich, da eine Sollposition auch dann erreicht werden kann, wenn ein Hindernis im Wege ist. Mit anderen Worten ist die Steuerungsvorrichtung 30 eingerichtet, die Antriebseinrichtung 60 derart anzusteuern, dass beim Verlagern in eine Sollposition ein Hindernis entgegen der Richtungsvorgabe eines Bedieners aktiv und autonom umfahren wird.

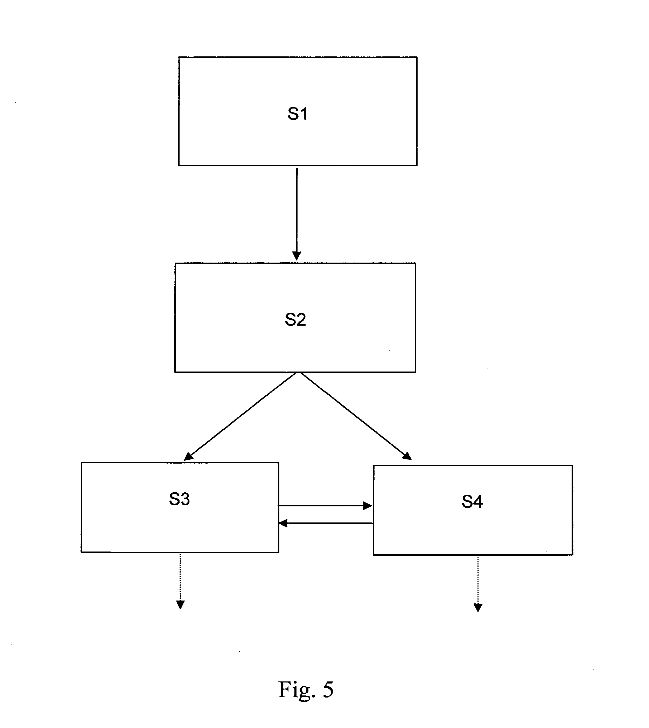

In der Figur 5 sind Verfahrensschritte eines Verfahrens zum Überwachen einer in einem Operationssaal angeordneten Stativvorrichtung hinsichtlich einer Kollision gezeigt. Das Verfahren umfasst mindestens drei Schritte, darunter den ersten Schritt S1, den zweiten Schritt S2 und den dritten Schritt S3 und/oder den vierten Schritt S4. Das Verfahren kann sowohl nach dem dritten Schritt S3 als auch nach dem vierten Schritt S4 beendet werden. Mit anderen Worten kann wahlweise der weitere dritte Schritt S3 und/oder der weitere vierte Schritt S4 vorgesehen sein. Der erste Schritt S1 entspricht bevorzugt einem Erfassen einer Relativposition mindestens eines Tragarms der Stativvorrichtung und/oder einer medizintechnischen Einrichtung der Stativvorrichtung, jeweils relativ zur Umgebung, insbesondere relativ zu weiteren im Operationssaal angeordneten Hindernissen, mittels mindestens einer Sensoreinrichtung. Allgemein kann auch zunächst nur ein Hindernis in einem Aktionsradius der Stativvorrichtung erfasst werden. Der zweite Schritt S2 entspricht einem Auswerten einer bzw. der erfassten Relativposition mittels einer Steuerungseinrichtung. Der dritte Schritt S3 entspricht einem Anzeigen einer zu einer Kollision mit den Hindernissen führenden Relativbewegung in Abhängigkeit der erfassten Relativposition mittels mindestens einer Anzeigeeinrichtung, insbesondere einer an der Stativvorrichtung angeordneten Anzeigeeinrichtung. Das Anzeigen der kritischen Relativbewegung kann z.B. durch irgendein Warnsignal erfolgen, welches nicht notwendigerweise optischer Art sein muss. Der vierte Schritt S4 entspricht einem aktiven Verhindern einer Kollision, insbesondere durch Ansteuern einer Bremseinrichtung und/oder einer Antriebseinrichtung der Stativvorrichtung.

### Bezugszeichenliste

- 1: Stativvorrichtung, insbesondere Deckenstativvorrichtung
- 10: Tragsystem
- 11: Montageeinrichtung, insbesondere Deckenflansch
- 12.1: (erstes) Drehgelenk
- 12.2: zweites Drehgelenk
- 12.3: drittes Drehgelenk
- 13: (erster) Tragarm
- 14: zweiter Tragarm
- 20: medizintechnische Einrichtung, insbesondere Versorgungskonsole
- 21: Träger, insbesondere Konsolenrohr
- 22: Griff
- 23: Taste
- 30: Steuerungsvorrichtung
- 31: Sensoreinrichtung, insbesondere Infrarotsensor
- 32: Sensoreinrichtung, insbesondere Ultraschallsensor
- 40: Anzeigeeinrichtung
- 41: Ausgabeelement, insbesondere visuelles und/oder akustisches Ausgabeelement
- 42: Ausgabeelement, insbesondere haptisches Ausgabeelement
- 50: Bremseinrichtung
- 51: (erste) Bremse
- 52: zweite Bremse
- 60: Antriebseinrichtung
- 61: (erster) Drehantrieb
- 62: zweiter Drehantrieb

- S1: erster Schritt
- S2: zweiter Schritt
- S3: dritter Schritt
- S4: vierter Schritt

## Patentansprüche

1. Stativvorrichtung (1) zur Anordnung in einem Operationssaal und zum örtlichen Verlagern einer medizintechnischen Einrichtung (20) im Operationssaal, umfassend
- die medizintechnische Einrichtung (20);
- ein Tragsystem (10) umfassend eine Montageeinrichtung (11) und mindestens einen daran bewegbar, insbesondere schwenkbar in einem Drehgelenk (12.1, 12.2, 12.3), gelagerten Tragarm (13, 14), wobei die medizintechnische Einrichtung am Tragarm befestigt ist und in einem Aktionsradius entsprechend dem Bewegungsfreiheitsgrad des Tragsystems verlagerbar ist;
**dadurch gekennzeichnet, dass** die Stativvorrichtung (1) eingerichtet ist, mindestens ein Hindernis im Aktionsradius der Stativvorrichtung zu erfassen und eine mögliche Kollision mit dem Hindernis anzuzeigen und/oder zu verhindern.

2. Stativvorrichtung nach Anspruch 1, **dadurch gekennzeichnet, dass** die Stativvorrichtung mindestens eine Sensoreinrichtung (31, 32) zum Erfassen der Relativposition des mindestens einen Tragarms (13, 14) und/oder der medizintechnischen Einrichtung (20) sowie eine mit der mindestens einen Sensoreinrichtung verbundene Steuerungseinrichtung (30) zum Auswerten der erfassten Relativposition aufweist.

3. Stativvorrichtung nach Anspruch 2, **dadurch gekennzeichnet, dass** die Steuerungseinrichtung (30) mit einer Anzeigeeinrichtung (40) zum Anzeigen einer zu einer Kollision mit dem Hindernis führenden Relativbewegung verbunden ist.

4. Stativvorrichtung nach Anspruch 2 oder 3, **dadurch gekennzeichnet, dass** die Stativvorrichtung mindestens eine Bremseinrichtung (50) aufweist, welche mit der Steuerungseinrichtung (30) verbunden ist, und welche mittels der Steuerungseinrichtung derart ansteuerbar ist, dass eine Bewegung des Tragsystems (10) bei einer zu einer Kollision führenden Relativbewegung zumindest teilweise blockierbar ist.

5. Stativvorrichtung nach einem der Ansprüche 2 bis 4, **dadurch gekennzeichnet, dass** die Stativvorrichtung (1) mindestens eine Antriebseinrichtung (60) aufweist, welche mit der Steuerungseinrichtung (30) verbunden ist, und welche mittels der Steuerungseinrichtung derart ansteuerbar ist, dass eine Bewegung des Tragsystems (10) bei einer zu einer Kollision führenden Relativbewegung beeinflussbar ist.

6. Stativvorrichtung nach einem der vorhergehenden Ansprüche, **dadurch gekennzeichnet, dass** die Stativvorrichtung eine Mehrzahl von Sensoreinrichtungen (31, 32) aufweist, wobei die Sensoreinrichtungen (31, 32) am mindestens einen Tragarm (13, 14) und/oder an der medizintechnischen Einrichtung (20) angeordnet sind.

7. Stativvorrichtung nach einem der vorhergehenden Ansprüche, **dadurch gekennzeichnet, dass** die Stativvorrichtung mindestens eine Sensoreinrichtung (31, 32) aus der Gruppe umfassend die folgenden Sensoreinrichtungen aufweist: Infrarotsensor, Ultraschallsensor, kapazitiver Sensor, induktiver Sensor, Radarsensor.

8. Stativvorrichtung nach einem der vorhergehenden Ansprüche, **dadurch gekennzeichnet, dass** die Stativvorrichtung mindestens zwei Tragarme (13, 14) aufweist, an welchen jeweils eine Mehrzahl von Sensoreinrichtungen (31, 32), insbesondere zwei, drei oder vier Sensoreinrichtungen, angeordnet sind, bevorzugt beidseitig und gegenüberliegend an Seitenflächen des jeweiligen Tragarms (13, 14).

9. Stativvorrichtung nach einem der vorhergehenden Ansprüche, **dadurch gekennzeichnet, dass** jeweils mindestens eine Sensoreinrichtung (31) am mindestens einen Tragarm (13, 14) angeordnet ist, wobei der Tragarm zumindest schwenkbar gelagert ist, und wobei die mindestens eine Sensoreinrichtung (31) eingerichtet ist, einen Erfassungsbereich zu überwachen, welcher in einer Erstreckungsebene des Tragarms (13, 14) einen größeren Erfassungswinkel aufweist als in einer Ebene senkrecht zur Erstreckungsebene.

10. Stativvorrichtung nach einem der vorhergehenden Ansprüche, **dadurch gekennzeichnet, dass** die mindestens eine Sensoreinrichtung ein Infrarotsensor (31) ist.

11. Stativvorrichtung nach einem der vorhergehenden Ansprüche, **dadurch gekennzeichnet, dass** die Stativvorrichtung höhenverstellbar ist, wobei die mindestens eine Sensoreinrichtung (32) an der medizintechnischen Einrichtung angeordnet ist, und wobei die Sensoreinrichtung eingerichtet ist, einen Erfassungsbereich zu überwachen, welcher kegelförmig ist, insbesondere kegelförmig mit einem Öffnungswinkel größer 45°, bevorzugt zwischen 60° und 90°, weiter bevorzugt zwischen 70° und 85°.

12. Stativvorrichtung nach einem der vorhergehenden Ansprüche, **dadurch gekennzeichnet, dass** die mindestens eine Sensoreinrichtung ein an einer Unterseite der medizintechnischen Einrichtung (20) angeordneter Ultraschallsensor (32) ist.

13. Stativvorrichtung nach einem der vorhergehenden Ansprüche, **dadurch gekennzeichnet, dass** die Stativvorrichtung eine Anzeigeeinrichtung (40) mit mindestens einem Anzeigeelement (41, 42) aufweist, welches zumindest am Tragsystem angeordnet ist und eingerichtet ist, ein akustisches und/oder visuelles Signal auszugeben.

14. Stativvorrichtung nach einem der vorhergehenden Ansprüche, **dadurch gekennzeichnet, dass** die Stativvorrichtung eine Anzeigeeinrichtung (40) mit mindestens einem Anzeigeelement (41, 42) aufweist, welches zumindest an der medizintechnischen Einrichtung, insbesondere an einem Griff und/oder einer Taste, angeordnet ist und eingerichtet ist, ein haptisches Signal, insbesondere ein Vibrieren, auszugeben.

15. Verfahren zum Überwachen einer in einem Operationssaal angeordneten Stativvorrichtung hinsichtlich einer Kollision, insbesondere einer Stativvorrichtung (1) nach einem der vorhergehenden Ansprüche, **gekennzeichnet durch** die Schritte:
- Erfassen eines Hindernisses in einem Aktionsradius der Stativvorrichtung (1) mittels mindestens einer Sensoreinrichtung (31, 32);
- Auswerten einer Relativposition der Stativvorrichtung relativ zum Hindernis mittels einer Steuerungseinrichtung (30);
- Anzeigen einer zu einer Kollision mit dem Hindernis führenden Relativbewegung in Abhängigkeit der Relativposition mittels mindestens einer Anzeigeeinrichtung (40), insbesondere einer an der Stativvorrichtung angeordneten Anzeigeeinrichtung; und/oder
- aktives Verhindern einer Kollision mit dem Hindernis mittels der Steuerungseinrichtung (30), insbesondere **durch** Ansteuern einer Bremseinrichtung (50) und/oder einer Antriebseinrichtung (60) der Stativvorrichtung.
